# FASCICULE DE BREVET EUROPEEN

(11) **EP 3 592 361 B1**
(45) Date de publication et mention de la délivrance du brevet: **13.09.2023**
(21) Numéro de dépôt: 18712981.2
(22) Date de dépôt: 09.03.2018
(51) Int. Cl.: A61K 31/662, A61P 29/00, A61P 27/02

(54) **DÉRIVÉS AMINOPHOSPHINIQUES POUR LA PRÉVENTION ET LE TRAITEMENT DE L'INFLAMMATION OCULAIRE**
AMINOPHOSPHINDERIVATE ZUR PRÄVENTION UND BEHANDLUNG VON AUGENENTZÜNDUNGEN
AMINOPHOSPHINIC DERIVATIVES FOR PREVENTING AND TREATING OCULAR INFLAMMATION

(30) Priorité: 09.03.2017 FR 1751912; 28.09.2017 FR 1759005
(43) Date de publication de la demande: 15.01.2020
(73) Titulaire: IACTA Pharmaceuticals, Inc., Guaynabo, 00968 (PR)
(72) Inventeur: PORAS, Hervé, 78450 Villepreux (FR); WURM, Michel, 63130 Royat (FR); MELIK PARSADANIANTZ, Stéphane, 94130 Nogent sur Marne (FR); REAUX-LE GOAZIGO, Annabelle, 94700 Maison Alfort (FR)
(74) Mandataire: Regimbeau
(86) Numéro de dépôt international: PCT/FR2018/050545
(87) Numéro de publication internationale: WO 2018/162860

(56) Documents cités:
- WO-A1-98/18803
- WO-A1-2010/010106
- WO-A1-2017/093322
- US-A1- 2009 111 794
- ELISABETH BONNARD ET AL: "Long-lasting oral analgesic effects of N -protected aminophosphinic dual ENKephalinase inhibitors (DENKIs) in peripherally controlled pain", PHARMACOLOGY RESEARCH & PERSPECTIVES, vol. 3, no. 2, 1 mars 2015 (2015-03-01), page e00116, XP055382976, GB ISSN: 2052-1707, DOI: 10.1002/prp2.116 cité dans la demande
- THOLANDER F ET AL: "Structure-Based Dissection of the Active Site Chemistry of Leukotriene A4 Hydrolase: Implications for M1 Aminopeptidases and Inhibitor Design", CHEMISTRY AND BIOLOGY, CURRENT BIOLOGY, LONDON, GB, vol. 15, no. 9, 22 septembre 2008 (2008-09-22), pages 920-929, XP025533970, ISSN: 1074-5521, DOI: 10.1016/J.CHEMBIOL.2008.07.018 [extrait le 2008-09-19] cité dans la demande

## Description

La présente invention se rapporte à la prévention et au traitement de l'inflammation oculaire.

L'inflammation ou la lésion des nerfs augmente les concentrations locales d'enképhalines au travers de divers mécanismes, comme la migration des cellules immunitaires contenant ces peptides opioïdes endogènes vers le site de la blessure (Hassan et al., 1993, Neuroscience, 55, 185-195), la libération d'enképhalines par les lymphocytes activés par des substances inflammatoires (chemokines, interleukines, LTB4, etc ...) (Machelska, 2007, Neuropeptides, 41, 355-363), la libération à partir de kératinocytes enflammés (Gabrilovac et al., 2004, Immunol. Lett., 91, 39-47) ou encore à partir de fibres nerveuses stimulées (Hassan et al., 1993, Neuroscience, 55, 185-195 ; Rittner et al., 2001, Anesthesiology, 95, 500-508). La régulation des récepteurs opioïdes se produit également dans les ganglions de la racine dorsale de la moelle épinière, avant que ces récepteurs soient efficacement transportés vers les terminaisons nerveuses périphériques (Hassan et al., 1993, Neuroscience, 55, 185-195).

Une autre famille de composés inflammatoires, les leukotrienes, sont synthétisés à partir de l'acide arachidonique, le précurseur des prostaglandines. Ce sont des agents très importants dans la réponse inflammatoire. L'activation de LTB4 génère la migration des cellules neutrophiles polynucléaires vers le site de l'inflammation mais aussi la production de radicaux libres oxygénés et l'expression de molécules d'adhésion. Les prostaglandines et les leukotrienes sont ainsi les médiateurs de différents aspects de l'inflammation.

Il a été montré que l'utilisation d'opioïdes et particulièrement de la morphine pouvait avoir un effet bénéfique sur l'inflammation oculaire. L'effet de l'administration topique de morphine sur le développement de l'inflammation a été étudié à l'aide d'un modèle de cautérisation de l'épithélium cornéen au nitrate d'argent chez le rat (Wenk et al., 2003, Pain, 105, 455-465). L'utilisation de morphine permet de retarder le développement d'un oedème du stroma (12h après traitement les cornées traitées semblent normales au contraire des non-traitées) et l'infiltration des cellules immunitaires (très peu de cellules immunitaires présentes).

L'augmentation apparente des récepteurs opioïdes fonctionnels à la suite d'une agression chimique renforce l'hypothèse de l'existence d'un système opioïde endogène dans la cornée activée par une lésion tissulaire ou une inflammation. Cet effet est probablement à la fois analgésique (anti-hyperalgésique) et anti-inflammatoire. L'activation d'un système opioïde endogène implique une disponibilité accrue des récepteurs et des ligands endogènes.

Les enképhalines (Met-enképhaline et Leu-enképhaline) sont des pentapeptides, opioïdes endogènes, initialement isolés dans le cerveau de mammifères (Hugues et al., 1975, Nature, 258, 577-580). Elles se lient principalement à deux classes de récepteurs, les récepteurs opioïdes mu et delta (Lord et al., 1977, Nature, 267, 495-499) dont les fonctions et les localisations sont différentes (Waksman et al., 1986, Proc. Natl. Acad. Sci., 83, 1523-1527).

D'autre part, on sait que les enképhalines (Tyr-Gly-Gly-Phe-Met et Tyr-Gly-Gly-Phe-Leu) sont physiologiquement inactivées par deux métallopeptidases à zinc, la néprilysine (EC 3.4.24.11, NEP) qui clive la liaison Gly³-Phe⁴ (Malfroy et al., 1978, Nature, 276, 523-526) et l'aminopeptidase N (EC 3.4.11.2, APN) qui coupe la liaison Tyr¹-Gly² de ces peptides. (Waksman et al., 1985, Eur. J. Pharmacol., 117, 233-243; revue dans Roques et al., 1993, Pharmacol. Rev., 45, 87-146).

Les dérivés aminophosphiniques de la présente invention, « vrais » inhibiteurs mixtes des enképhalinases, c'est-à-dire inhibant conjointement l'APN et la NEP, ont été décrit dans de précédents brevets et publications (WO98/18803; WO2010/010106; Chen et al., 2000, J. Med. Chem., 43, 1398-1408; Chen et al., 2001, J. Med. Chem., 44, 3523-3530; Le Guen et al., 2003, Pain, 104, 139-148; Bonnard et al., 2015, Pharmacol. Res. Persp., 3(2), e00116, doi: 10.1002/prp2.116) comme présentant une activité analgésique.

En revanche, l'utilisation de ces dérivés aminophosphiniques comme composés permettant de diminuer, par administration topique, l'inflammation oculaire n'a jamais été décrite ou suggérée.

Il n'a également jamais été démontré que des prodrogues d'inhibiteurs mixtes de NEP et d'APN pouvaient s'hydrolyser pour donner un composé capable d'atteindre sa cible au niveau de la cornée et permettant ainsi une réponse sur la diminution de l'inflammation. De la même manière, il n'a jamais été démontré que des prodrogues d'inhibiteurs de LTA4 hydrolase pouvaient s'hydrolyser pour donner un composé capable d'atteindre sa cible au niveau de la cornée et permettant ainsi une réponse sur la diminution de l'inflammation oculaire.

US2009/0111794A1 divulgue des composés diamines utiles en tant que modulateurs de LTA4 hydrolase pour le traitement de maladies, y compris l'inflammation.

L'un des objets de la présente invention est donc de fournir de nouveaux composés de type aminophosphiniques capables d'inhiber conjointement les deux activités enzymatiques (néprilysine et aminopeptidase N) responsables de la dégradation des enképhalines, ces derniers pouvant avoir une action dans le cadre de l'inflammation oculaire.

Or, les Inventeurs ont notamment démontré que le composé 1, composé de la présente invention, qui est une pro-drogue d'un inhibiteur de LTA4 hydrolase (Tholander et al., 2008, Chem. Biol., 15, 920-929) empêchant ainsi la formation de LTB4 (Bonnard et al., 2016, Pharma. Res. Per., 3(2), 2015, e00116, doi: 10.1002/prp2.116), génère, par son administration topique, un composé ayant une action inflammatoire dans la partie antérieure de l'oeil.

Ainsi, l'invention a plus particulièrement pour objet des composés répondant à la formule (I), ou un sel pharmaceutiquement acceptable de ces composés pour leur utilisation dans le traitement et/ou la prévention de l'inflammation oculaire :

(I) R₁-NH-CH(R₂)-P(=O)(OH)-CH₂-C(R₃)(R₄)-CONH-C(R₅)(R₆)-COOR₇

Dans laquelle :
R₁ représente
   - un hydrogène
   - un groupement (acyloxy)alkyl carbamate -C(=O)-O-C(R)(R')-OC(=O)-R" dans lequel R et R' représentent, indépendamment l'un de l'autre, un hydrogène, un groupement alkyle et R" représente un groupement alkyle,
R₂ représente :
   - une chaîne hydrocarbonée, saturée ou insaturée, linéaire ou ramifiée comportant de 1 à 6 atomes de carbone,
R₃ et R₄ représentent indépendamment l'un de l'autre:
   - un hydrogène
   - un groupement phényle ou benzyle, éventuellement substitué sur le noyau phényle par :
      * 1 à 5 atomes d'halogènes notamment le fluor ou le brome
      * un radical OH, SH, OR" ou SR", R" ayant la même définition que précédemment
      * un groupement amino éventuellement mono- ou di-substitué par un groupement aliphatique, cyclique ou linéaire, de 1 à 6 atomes de carbone
      * un groupement trifluorométhyle
      * un groupement aromatique ou hétéroaromatique à 5 ou 6 atomes
   - un groupement hétéroaromatique à 5 ou 6 atomes, contenant 1 ou 2 hétéroatome(s) pris parmi l'oxygène, l'azote ou le soufre, les atomes de soufre et d'azote pouvant être oxydés sous forme de S-oxyde ou de N-oxyde
   - un méthylène substitué par un hétérocycle à 5 ou 6 atomes, aromatique ou saturé, l'hétéroatome étant un oxygène, un azote ou un soufre, les atomes d'azote et de soufre pouvant être oxydés sous forme N-oxyde ou de S-oxyde
R₃ et R₄ ne représentent pas simultanément un atome d'hydrogène,
R₅ et R₆ représentent indépendamment l'un de l'autre
   - un atome d'hydrogène
   - une chaîne hydrocarbonée saturée ou insaturée, linéaire ou ramifiée de 1 à 6 atomes de carbone,
R₇ représente
   - un hydrogène
   - un radical CH₂COOR‴ ou CH(CH₃)COOR‴, R‴ représentant :
      *une chaîne hydrocarbonée saturée de 1 à 6 atomes de carbone, éventuellement substituée par un groupement alkoxy de C₁ à C₃,
      *un groupement cycloalkyle de C₅ à C₈
      *un groupement phényle, benzyle, hétéroaromatique ou alkyl hétéroaromatique
   - un groupement CH(R)O-C(O)OR' ou CH(R)OC(O)R' dans lesquels R et R' ont les mêmes définitions que précédemment.

L'inflammation oculaire peut être notamment due à un traumatisme, une infection et/ou une maladie auto-immune. Les composés selon l'invention sont avantageusement utilisés pour améliorer le processus de guérison et/ou de cicatrisation oculaire, et ainsi prévenir par exemple l'apparition d'une kératopathie toxique, de problèmes au niveau de l'épithélium ou de cicatrices permanentes.

Les composés selon l'invention peuvent notamment être utilisés pour prévenir ou traiter une inflammation de la cornée, de la sclère, de l'uvée. Ainsi, les composés selon l'invention sont avantageusement utilisés pour prévenir ou traiter une inflammation oculaire liée à une kératite, une sclérite, une épisclérite, une uvéite, une cataracte, une synéchie, un oedème maculaire, un décollement de la rétine, une hypertension oculaire et la dégénérescence du nerf optique causée par le glaucome. En particulier, les composés selon l'invention sont également utilisés pour prévenir la formation de nouveaux vaisseaux (néovascularisation).

La présente invention a également trait à une composition ophtalmique comprenant un composé de formule (I) de l'invention, en particulier collyre, pommade ophtalmique, gel ophtalmique, ou insert ophtalmique, notamment pour son utilisation dans le traitement et/ou la prévention de l'inflammation oculaire.

Dans la présente invention, le patient souffrant d'inflammation oculaire est typiquement un animal, de préférence un animal, avantageusement il s'agit d'un homme.

### Inflammation oculaire

L'inflammation est une réponse physiologique de l'organisme à une agression qui peut être d'origines diverses: traumatisme; infection; maladie auto-immune. Au niveau de l'oeil, l'inflammation provoque notamment une rupture des barrières qui isolent l'œil de la circulation générale, permettant l'entrée de protéines et de cellules qui participent au processus inflammatoire et ont un impact sur la cicatrisation.

De nombreux anesthésiques ophtalmiques topiques ralentissent le processus de guérison, ce qui peut conduire à des complications telles qu'une kératopathie toxique, des problèmes au niveau de l'épithélium ou des cicatrices permanentes (Willis et Laibson, 1970, Can. J. Ophthalmol., 5, 239-243; Burstein et Klyce, 1977, Invest. Ophthalmol. Vis. Sci., 16 (10), 899-911; Rocha et al., 1995, Can. J. Ophthalmol., 30(4), 198-202). Par conséquent, les médecins hésitent souvent à prescrire des anesthésiques topiques, même pour les douleurs oculaires graves (Reiser et Laibson, 1989, Ophthalmic Surg., 20, 72-73).

L'inflammation peut toucher la cornée et la sclère à la surface de l'œil; l'uvée composée de l'iris, du corps ciliaire et de la choroïde; et à l'origine du nerf optique, la rétine et la papille. Avantageusement, l'inflammation oculaire touche la sclère à la surface de l'œil; l'uvée composée de l'iris, du corps ciliaire et de la choroïde; et à l'origine du nerf optique, la rétine et la papille. Cette inflammation oculaire peut être responsable d'opacité et de lésions de la cornée (kératite); de lésions de la sclère (sclérite et épisclérite); d'uvéite avec pour complication une opacification du cristallin (cataracte); des adhérences de l'iris au cristallin (synéchies); un oedème maculaire; un décollement de la rétine; une hypertension oculaire et la dégénérescence du nerf optique causée par le glaucome; la formation de nouveaux vaisseaux (néovascularisation); etc.

Dans un mode de réalisation particulier, l'inflammation oculaire n'est pas nécessairement causée par une kératite.

### Composés selon l'invention :

Dans la présente invention, on entend par « pharmaceutiquement acceptable » ce qui est utile dans la préparation d'une composition pharmaceutique qui est généralement sûr, non toxique et ni biologiquement ni autrement non souhaitable et qui est acceptable pour une utilisation vétérinaire de même que pharmaceutique humaine.

Par « sels pharmaceutiquement acceptables » d'un composé, on entend désigner dans la présente invention des sels qui sont pharmaceutiquement acceptables, comme défini ici, et qui possèdent l'activité pharmacologique souhaitée du composé parent. Dans le cadre de la présente invention, il s'agit de sels d'addition obtenus avec une base minérale ou organique. Ainsi, le sel formé correspond :
- soit au remplacement d'un proton acide par un ion métallique, par exemple un ion de métal alcalin (Na⁺, K⁺ ou Li⁺ par exemple), un ion de métal alcalino-terreux (comme Ca²⁺ ou Mg²⁺) ou un ion d'aluminium,
- soit à la coordination de ce proton acide avec une base organique ou inorganique. Les bases organiques acceptables comprennent des amines telles que l'ammoniaque, la diéthanolamine, l'éthanolamine, la N-méthylglucamine, la triéthanolamine, la triéthylamine, la trométhamine et similaires. Les bases inorganiques acceptables comprennent l'hydroxyde d'aluminium, l'hydroxyde de calcium, l'hydroxyde de lithium (lithine), l'hydroxyde de potassium (potasse), le carbonate de sodium et l'hydroxyde de sodium (soude).

Avantageusement, les sels pharmaceutiquement acceptables des composés de l'invention seront des sels d'addition obtenus avec une base minérale ou organique pharmaceutiquement acceptable, telle que la lithine, la soude, la potasse, l'ammoniaque, une amine tertiaire de formule NRₐR_{b}R_{c}, où Rₐ, R_{b} et R_{c} représentent, indépendamment les uns de autres, un groupe alkyle tel que défini ci-dessous, comme la triéthylamine, ou encore un aminoacide basique tel que la lysine ou l'arginine et leurs dérivés.

Par « insaturé », on entend, au sens de la présente invention, que la chaîne hydrocarbonée comprend une ou plusieurs insaturation(s). Par « insaturation », on entend, au sens de la présente invention, une double ou une triple liaison.

Par « atome d'halogène », on entend, au sens de la présente invention, un atome de fluor, de chlore, de brome ou d'iode. Avantageusement, il s'agit d'un atome de fluor, de brome ou de chlore. Encore avantageusement, il s'agit d'un atome de fluor ou de brome, et de préférence de fluor.

Par groupe « amino », on entend, au sens de la présente invention, un groupe de formule -NR*R**, où R* et R** représentent, indépendamment l'un de l'autre, un atome d'hydrogène ou un groupe hydrocarboné saturé ou insaturé, linéaire, ramifié ou cyclique, comportant de 1 à 6, de préférence de 1 à 4, atomes de carbone, ou R* et R** forment ensemble, avec l'atome d'azote qui les porte, un hétérocycle à 5 ou 6 chaînons, saturé ou non, et ne comportant pas d'autre hétéroatome que l'azote qui porte les deux radicaux R* et R**. En particulier, le groupe amino peut être un groupe -NH₂, -NHMe, -NHEt, -NHPr, NHiPr, -NHBu, -NHiBu, -NHtBu, pipéridinyle ou pyrrolidinyle.

Par groupement « aromatique », on entend, au sens de la présente invention, un groupement aromatique, comportant de préférence de 5 à 10 atomes de carbone, sauf mention contraire, et comprenant un ou plusieurs cycles accolés, comme par exemple un groupement phényle ou naphtyle. Avantageusement, il s'agit du phényle.

Par groupement « hétéroaromatique », on entend, au sens de la présente invention, tout groupe aromatique tel que défini ci-dessus dans lequel un ou plusieurs atome(s) de carbone a(ont) été remplacé(s) par un ou plusieurs hétéroatome(s), avantageusement 1 à 4 et, encore plus avantageusement 1 à 2, tels que par exemple des atomes de soufre, azote ou oxygène, les atomes de soufre et d'azote pouvant être éventuellement oxydé sous forme de S-oxyde ou de N-oxyde. Des exemples de groupes hétéroaromatique sont les groupes furyle, thiényle, pyrrolyle, pyridinyle, pyrimidyle, pyrazolyle, imidazolyle, tétrazolyle ou encore indanyle.

Par « cycle hétéroaromatique à 5 ou 6 atomes », on entend, au sens de la présente invention, un groupe hétéroaromatique tel que défini ci-dessus ne comportant qu'un seul cycle à 5 ou 6 atomes. Il s'agit notamment d'un groupe thiényle, pyrrolyle, pyridinyle, pyrimidyle, pyrazolyle, imidazolyle ou encore tétrazolyle.

Par « hétérocycle », on entend, au sens de la présente invention, un cycle hydrocarboné, avantageusement à 5 ou 6 atomes, dont un ou plusieurs atome(s) de carbone a(ont) été remplacé(s) par un ou plusieurs hétéroatome(s), avantageusement 1 à 4 et, encore plus avantageusement 1 à 2, tels que par exemple des atomes de soufre, azote ou oxygène, les atomes de soufre et d'azote pouvant être éventuellement oxydés sous forme de N-oxyde et de S-oxyde. Sauf mention contraire, ce cycle pourra être saturé ou aromatique. Dans le cas où le ou les hétéroatome(s) est (sont) choisi(s) parmi l'azote et le soufre, l'hétérocycle peut être en particulier un groupe : pipéridinyle, pyrrolidinyle, pyrrolyle, thiényle, pyrrazolyle, imidazolyle, pyridinyle, pyridazinyle, pyrimidinyle, pipérazinyle, thiadiazolyle, tétrahydrothiényle ou encore thiazolyle.

Par « alkyle », on entend, au sens de la présente invention, une chaîne hydrocarbonée saturée, linéaire ou ramifiée, comportant de 1 à 6 atomes de carbone, sauf mention contraire. Il s'agit en particulier des groupes méthyle, éthyle, n-propyle, isopropyle, n-butyle, iso-butyle, sec-butyle, tert butyle, n-pentyle, n-hexyle.

Par « cycloalkyle », on entend, au sens de la présente invention, un cycle hydrocarboné saturé comportant de 5 à 8 atomes de carbone, en particulier le groupe cyclohexyle, cyclopentyle ou cycloheptyle.

Par «alkylehétéroaromatique », on entend, au sens de la présente invention, un groupe hétéroaromatique tel que défini ci-dessus lié à la molécule par l'intermédiaire d'un groupe alkyle tel que défini ci-dessus. Il s'agit en particulier d'un groupe thiénylméthyle ou furylméthyle.

Dans une première variante, R₁ représente un groupement (acyloxy)alkyl carbamate - C(=O)-O-C(R)(R')-OC(=O)-R". En particulier, R₁ représente un groupement -C(=O)-O-CHMe-OC(=O)-CHMe₂.

Dans une deuxième variante, R₁ représente un atome d'hydrogène.

De manière également avantageuse, le radical R₂ représente une chaîne hydrocarbonée, saturée, linéaire ou ramifiée, comportant de 1 à 4 atomes de carbone. De préférence, le radical R₂ représente un groupe méthyle.

Selon une variante avantageuse de l'invention, R₃ représente un atome d'hydrogène et R₄ est tel que défini précédemment. Avantageusement, R₃ représente un atome d'hydrogène et R₄ représente un groupe benzyle éventuellement substitué par 1 à 5 atome(s) d'halogène tel(s) que le fluor ou le brome, un phényle ou un groupement hétéroaromatique à 5 ou 6 chaînons, de préférence R₄ représente groupe benzyle éventuellement substitué par un phényle (notamment en position para). En particulier, R₃ représente un atome d'hydrogène et R₄ représente un groupe benzyle substitué, en position para, par un atome d'halogène, tel qu'un atome de brome, ou par un phényle.

De manière également avantageuse, le radical R₄ représente un groupe benzyle éventuellement substitué par 1 à 5 atome(s) d'halogène tel(s) que le fluor ou le brome, un phényle ou un groupement hétéroaromatique à 5 ou 6 chaînons, de préférence R₄ représente groupe benzyle éventuellement substitué par un phényle (notamment en position para).

De manière également avantageuse, le radical R₅ représente un atome d'hydrogène.

De manière également avantageuse, le radical R₆ représente un groupement alkyle tel qu'un groupement méthyle.

De manière également avantageuse, le radical R₇ représente un atome d'hydrogène ou un benzyle.

Selon une variante avantageuse de l'invention, les radicaux ont la signification suivante:
- R₁ représente un groupe -C(=O)-O-C(R)(R')-OC(=O)-R" dans lequel R représente un atome d'hydrogène et R' et R" représentent un groupe alkyle ;
- R₂ représente un groupe alkyle,
- R₃ représente un atome d'hydrogène ;
- R₄ représente un groupe benzyle substitué en position para par un atome d'halogène (brome) ou par un phényle ;
- R₅ représente un atome d'hydrogène ;
- R₆ représente un groupe alkyle ;
- R₇ représente un atome d'hydrogène.

Selon un mode de réalisation particulier, le composé de l'invention est choisi parmi les composés suivants :
Ester benzylique de l'acide 2-(2-Biphényl-4-ylméthyl-3-{hydroxy-[1-(1-isobutyryloxy-éthoxycarbonylamino)-éthyl]-phosphinoyl}-propionylamino)-propionique
Acide 2-(2-Biphényl-4-ylméthyl-3-{hydroxy-[1-(1-isobutyryloxy-éthoxycarbonylamino)-éthyl]-phosphinoyl}-propionylamino)-propionique
Ester éthylique de l'acide 2-(2-Biphényl-4-ylméthyl-3-{hydroxy-[1-(1-isobutyryloxy-éthoxycarbonylamino)-éthyl]-phosphinoyl}-propionylamino)-propionique
Ester éthoxycarbonyloxy de l'acide 2-(2-Biphényl-4-ylméthyl-3-{hydroxy-[1-(1-isobutyryloxy-éthoxycarbonylamino)-éthyl]-phosphinoyl}-propionylamino)-propionique
Ester benzylique de l'acide 2-(2-(4-Bromo-benzyl)-3-{hydroxy-[1-(1-isobutyryloxy-éthoxycarbonylamino)-éthyl]-phosphinoyl}-propionylamino)-propionique
Acide 2-(2-(4-Bromo-benzyl)-3-{hydroxy-[1-(1-isobutyryloxy-éthoxycarbonylamino)-éthyl]-phosphinoyl}-propionylamino)-propionique
Ester benzylique de l'acide 2-(2-Biphényl-4-ylméthyl-3-{hydroxy-[1-(amino)-éthyl]-phosphinoyl}-propionylamino)-propionique
Acide 2-(2-Biphényl-4-ylméthyl-3-{hydroxy-[1-(amino)-éthyl]-phosphinoyl}-propionylamino)-propionique
Ester éthylique de l'acide 2-(2-Biphényl-4-ylméthyl-3-{hydroxy-[1-(1amino)-éthyl]-phosphinoyl}-propionylamino)-propionique
Ester éthoxycarbonyloxy de l'acide 2-(2-Biphényl-4-ylméthyl-3-{hydroxy-[1-(amino)-éthyl]-phosphinoyl}-propionylamino)-propionique
Ester benzylique de l'acide 2-(2-(4-Bromo-benzyl)-3-{hydroxy-[1-(amino)-éthyl]-phosphinoyl}-propionylamino)-propionique
Acide 2-(2-(4-Bromo-benzyl)-3-{hydroxy-[1-(amino)-éthyl]-phosphinoyl}-propionylamino)-propionique
Ester éthylique de l'acide 2-(2-(4-Bromo-benzyl)-3-{hydroxy-[1-(amino)-éthyl]-phosphinoyl}-propionylamino)-propionique.

De préférence, il s'agit du composé 1 :

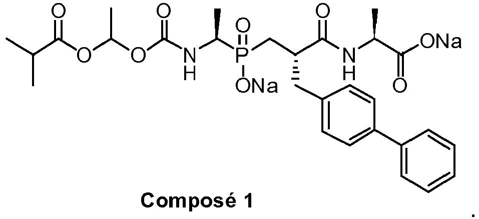

Les composés de formule (I) ont été décrits comme inhibiteurs doubles de l'APN et de la NEP et présentent des activités analgésiques dans de nombreux modèles de douleurs centrales ou périphériques après une administration intraveineuse ou orale (Chen et al., 2000, J. Med. Chem., 43, 1398-1408; Bonnard et al., 2015, Pharmacol. Res. Persp., 3(2), e00116, doi: 10.1002/prp2.116).

Les composés de formule (I) peuvent être synthétisés, par exemple, par des méthodes décrites dans : FR 2 755 135 et FR 2 934 267 . Le composé 1, par exemple, peut être synthétisé comme décrit dans Chen et al., 2000, J. Med. Chem., 43, 1398-1408.

Les composés de formule (I) sont formulés en accord avec des méthodes décrites par l'homme de l'art, en particulier pour la voie d'administration désirée.

En particulier, les composés sont formulés sous la forme d'une composition ophtalmique, en particulier de collyres, pommades ophtalmiques, gels ophtalmiques, ou inserts ophtalmiques.

De manière préférentielle, les compositions administrées de la présente invention seront formulées comme solutions, suspensions ou autre dosage pour administration topique, en particulier une administration oculaire. Par conséquent, de telles compositions sont formulées de sorte à respecter : une bonne tolérance (en particulier pH acceptable), une osmolarité physiologique. Il est en outre préférable que les compositions soient stériles et formulées de sorte à éviter une contamination bactérienne en cours d'utilisation. Les solutions ophtalmiques sont avantageusement limpides, caractérisées par une absence de particules. Les suspensions sont avantageusement caractérisées par une taille des particules inférieure à 25 µm. A titre de formulations galéniques pour administration oculaire, on peut notamment citer :
- Les collyres : sous la forme de solutions, suspensions, émulsions stériles, contenant un ou plusieurs principes actifs. On préfère les solvants ou mélange de solvants aqueux ou hydrosolubles. Les collyres peuvent être conditionnés en emballage multidoses ou unidoses.
- Les pommades ophtalmiques, c'est-à-dire des préparations semi-solides, stériles, destinées à être appliquées sur la conjonctive, contenant un ou plusieurs principes actifs et des excipients appropriés (vaseline, paraffine liquide).
- Les gels ophtalmiques, c'est-à-dire des préparations semi-solides, stériles, destinées à être appliquées sur la conjonctive, contenant un ou plusieurs principes actifs et des excipients appropriés. L'excipient est avantageusement un polymère hydrophile gélifiant en présence d'eau (carbomère, carbopol^{®}, acide polyacrylique).
- Les Inserts ophtalmiques, c'est-à-dire des préparations solides ou semi-solides stériles, destinées à être insérées dans le sac conjonctival. Ils sont en général constitués d'un réservoir de principe actif encastré dans une matrice entourée d'une membrane permettant de contrôler la libération. Le principe actif est libéré progressivement.

Les solutions aqueuses seront utilisées de préférence car elles sont plus facilement formulées, et il est également plus facile pour un patient de s'administrer une telle composition à l'aide d'une instillation de 1 ou 2 gouttes de la solution dans l'œil affecté. Malgré tout, la composition pourra également être une suspension, un gel visqueux ou semi-visqueux ou d'autres types de compositions solides ou semi-solides.

Le véhicule utilisé de préférence pour les formulations ophtalmiques de la présente invention est l'eau milliQ, et de manière préférentielle une solution saline physiologique. Afin d'éviter toute dérive du pH pendant le stockage, le pH d'une telle solution sera de préférence maintenu entre 5,0 et 8, notamment entre 5,5 et 8,0, et de manière préférentielle entre 6,5 et 7,2, avec le tampon approprié tels que des tampons acétates, citrates, phosphates ou borates. Les formulations pourront également contenir les conventionnels, acceptable pharmaceutiquement, conservateurs, stabilisants et/ou composés favorisant la pénétration.

Ainsi, la composition ophtalmique est avantageusement une solution aqueuse, ayant avantageusement un pH allant de 5,0 à 8, notamment entre 5,5 et 8,0.

Les compositions administrées en accord avec les méthodes décrites dans la présente invention contiennent une quantité active pour une utilisation ophtalmique d'un composé de formule (I). Cela signifie une quantité suffisante pour prévenir ou soulager une inflammation oculaire. Généralement, les compositions décrites dans la présente invention contiendront de 0,01% à 3% (poids/volume) d'un composé de formule (I). De préférence, les compositions de la présente invention contiendront de 0,1 à 1% (poids/volume) d'un composé de formule (I). Ainsi, la composition ophtalmique comprend avantageusement de 0,01% à 3% en poids par volume dudit composé de formule (I), plus avantageusement de 0,1% à 1% en poids par volume.

La composition administrée pourra également contenir d'autres ingrédients variés tels que, mais non de manière exhaustive, des surfactants, des agents influant sur l'osmolarité, des tampons, des conservateurs, des co-solvants ou des agents augmentant la viscosité.

Différents composés influençant l'osmolarité peuvent être utilisés pour ajuster l'osmolarité d'une solution afin de se rapprocher de la composition des larmes naturelles. Par exemple, le chlorure de sodium, le chlorure de potassium, le chlorure de magnésium, le chlorure de calcium, le dextrose et/ou le mannitol peuvent être utilisés afin de se rapprocher de l'osmolarité physiologique (de manière générale de l'ordre de 150-450 mOsm et de manière préférentielle 250-350 mOsm).

Les agents conservateurs qui peuvent être utilisés dans les formulations ophtalmiques de la présente invention peuvent être, mais ne sont pas limités à, du chlorure de benzalconium, du chlorobutanol, du bromure de benzododecinium, du methyl parabène, du propyl parabène, du thimerosal, de l'acétate phenylmercurique et du nitrate phenylmercurique. De tels conservateurs sont généralement utilisés à une dose de 0,001 à 1,0% poids/volume.

Les agents agissant sur la viscosité qui peuvent être utilisés dans les formulations ophtalmiques de la présente invention peuvent être, mais ne sont pas limités à, des polyols monomériques, de la povidone, de l'hydroxypropylmethyl cellulose, des poloxamers, de la carboxymethyl cellulose, des carbomères ou de l'hydroxyethylcellulose, des dextrans comme le dextran 70, des protéines solubles dans l'eau comme la gélatine

Les agents agissant sur la pénétration qui peuvent être utilisés sont par exemple, des solvants organiques comme le dimethylsulfoxyde ou autres sulfoxydes, le dimethylacétamide et les pyrrolidones; certains composés amides d'amines hétérocycliques, des glycols (ex propyleneglycol); des carbonates de propylène; de l'acide oléique; des amines alkylées et autres sels ammonium dérivés; des agents de surface variés anioniques, cationiques ou non-ioniques etc ...

Dans un mode de réalisation préféré, une formulation de la présente invention comprend une cyclodextrine, telle que l'hydroxypropyl béta-cyclodextrine ou bien encore la sulfobutyl ether béta-cyclodextrine, ou du sodium de polystyrène sulfonate.

Ainsi, la composition ophtalmique comprend avantageusement en outre une cyclodextrine, telle que l'hydroxypropyl béta-cyclodextrine ou bien encore la sulfobutyl ether béta-cyclodextrine, ou du sodium de polystyrène sulfonate.

### DESCRIPTION DES FIGURES

Figure 1. Images des différentes sections cornéennes souris contrôles Cage 1 : scratch + LPS + PBS
Figure 2. Images des différentes sections cornéennes souris contrôles Cage 1: scratch + LPS + PBS
Figure 3. Images des différentes sections cornéennes souris contrôles Cage 2: scratch + LPS (2x) + PBS
Figure 4. Images des différentes sections cornéennes souris traitées Cage 3: scratch + LPS (1x) + Composé 1 (10 mM)
Figure 5. images des différentes sections cornéennes souris traitées Cage 3: scratch + LPS (1x) + Composé 1 (10 mM)
Figure 6. Images des différentes sections cornéennes souris traitées Cage 4: scratch + LPS (2x) + Composé 1 (10 mM)
Figure 7. Images des différentes sections cornéennes souris traitées Cage 4: scratch + LPS (2x) + Composé 1 (10 mM)
Figure 8. Photographie du dispositif expérimental d'imagerie de la surface oculaire (capsule Tomocap) utilisé dans les exemples ci-dessous.

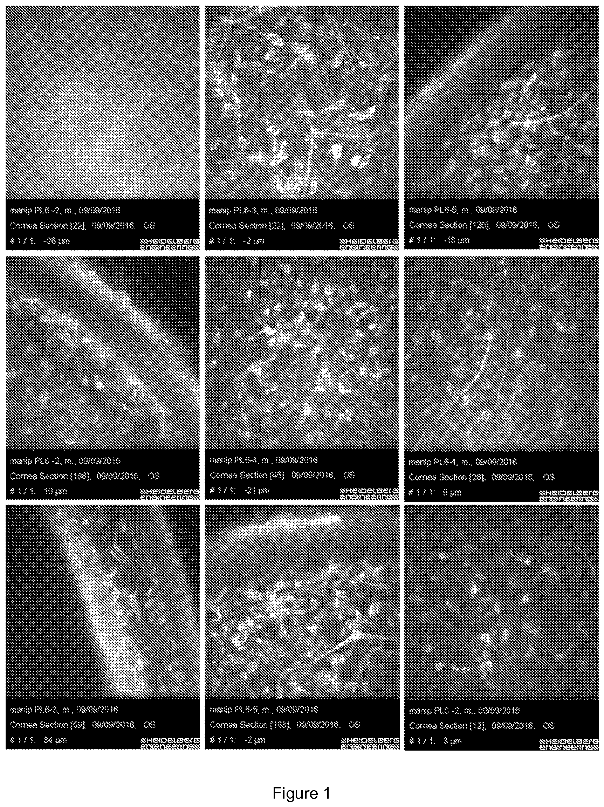

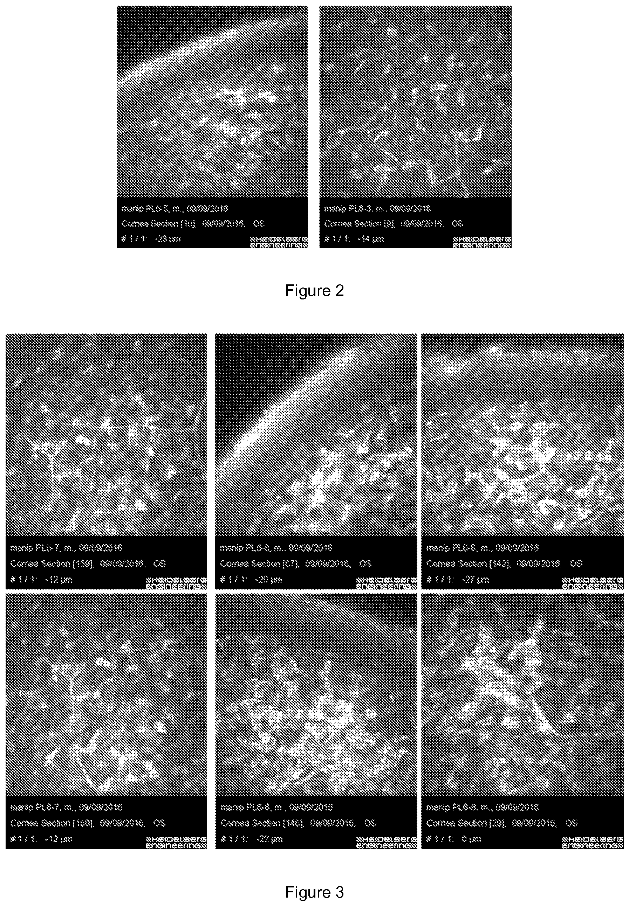

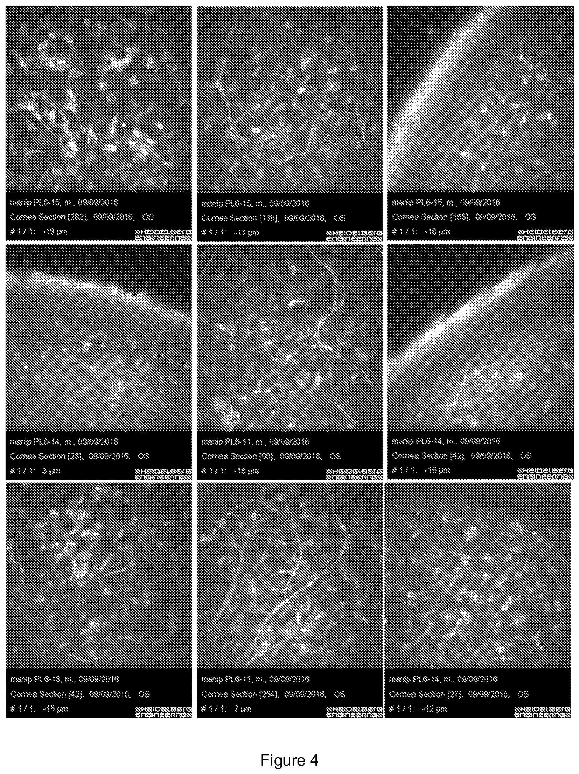

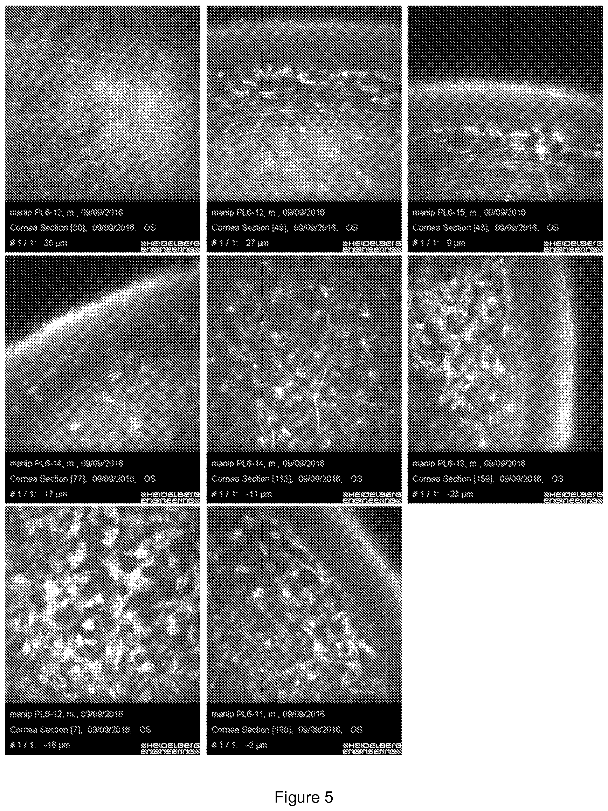

Les figures 1 et 2 servent de contrôle inflammatoire pour les figures 4 et 5.

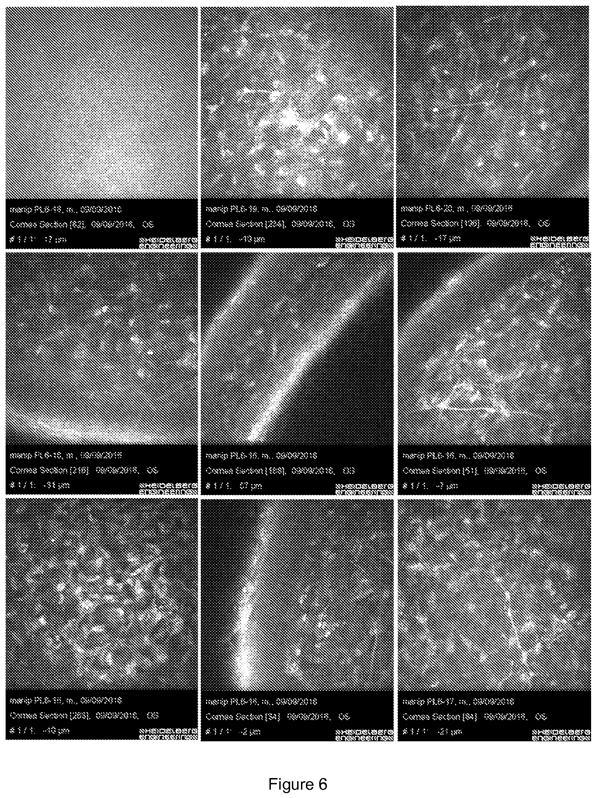

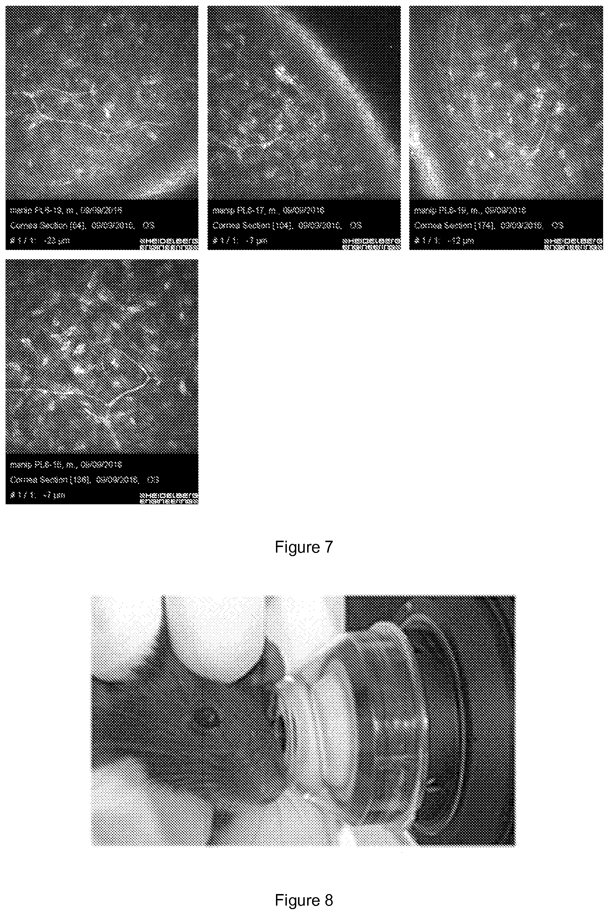

La figure 3 sert de contrôle inflammatoire pour les figures 6 et 7.

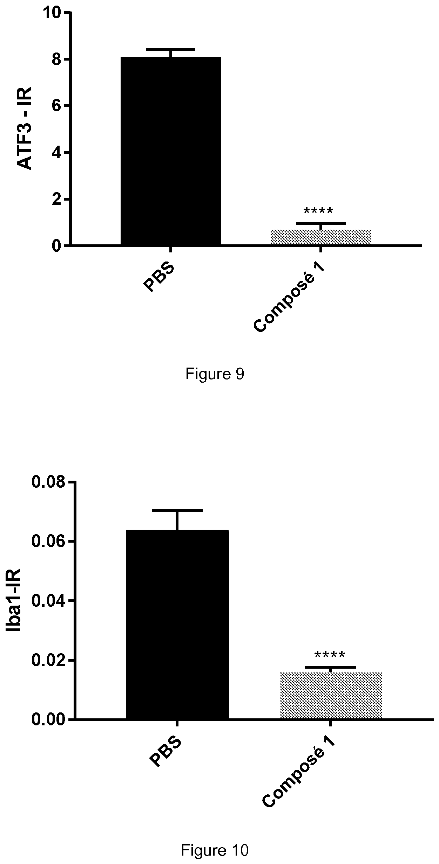

Figure 9. Expression du gène ATF3 («Activating transcription factor 3 ») dans le ganglion trigéminé. Dans un modèle d'inflammation au LPS, le traitement biquotidien pendant 5 jours par le composé 1, versus le groupe contrôle au PBS, réduit considérablement l'expression de ATF3, marqueur de stress neuronal lié à un processus inflammatoire.
Figure 10. Pourcentage relatif de la protéine Iba1 («lonized calcium binding adaptor molécule 1 ») dans le ganglion trigéminé. Dans un modèle d'inflammation au LPS, le traitement biquotidien pendant 5 jours par le composé 1, versus le groupe contrôle au PBS, réduit considérablement la coloration des cellules microgliales activées exprimant Iba-1, signe d'une production de macrophages.

### EXEMPLES

### Exemple 1 : activité pharmacologique du composé 1 (voir structure ci-dessus dans un modèle inflammatoire induit par du lipopolysaccharide (LPS, 50 µg)

Une lésion épithéliale cornéenne (abrasion appelée « scratch » dans les figures) est réalisée sur l'œil droit de souris mâles C57BI/6, âgées de 8 semaines, à l'aide d'une tréphine (diamètre 1,5 mm) sur animal anesthésié (isoflurane). Une goutte (10 µl) de LPS (50 µg) est déposée sur la zone lésée et est laissée au contact de la cornée jusqu'au réveil de l'animal.

L'expérimentateur s'assure du bon réveil de l'animal qui se fait dans les deux minutes qui suivent l'intervention.

Le premier traitement topique à l'aide d'une solution de PBS (tampon phosphate pH = 7,4) (1x) ou d'une solution de composé 1 (10 mM en composé 1 dans une solution de PBS (1x)) a été effectué 2h après l'instillation de LPS. Ce traitement est prolongé 2 fois par jour pendant 5 jours.

Quatre cages d'animaux sont étudiées :
Cage 1: Scratch LPS (instillation J1) + PBS 1x (n=5)
Cage 2: Scratch LPS (2x) (instillations J1 et J4) + PBS 1x (n=5)
Cage 3: Scratch LPS (instillation J1) + **composé 1** (10mM) (n=5)
Cage 4: Scratch LPS (2x) (instillations J1 et J4) + **composé 1** (10mM) (n=5)

L'inflammation oculaire des souris est évaluée au 5^{ème} jour à l'aide d'un microscope confocal *in vivo.* Celle-ci permet l'obtention d'images (définition cellulaire et intercellulaire) de la cornée comprenant l'épithélium cornéen, le plexus nerveux, le stroma et l'endothélium grâce à une capsule (Tomocap)/ module cornéen de l'appareil placée au contact de la cornée. Les changements morphologiques de l'ensemble des cellules constituant la cornée et les nerfs cornéens sont facilement observables et quantifiables. L'ensemble des animaux est anesthésié par injection intrapéritonéale (kétamine 100mg/kg et xylazine 10mg/kg). Du gel Lubrithal^{®} est déposé sur l'œil non traité.

Le dispositif expérimental utilisé dans cette procédure est identique à celui utilisé en routine en clinique humaine. Les animaux sont anesthésiés et les deux yeux reçoivent du gel Lubrithal^{®}. Le dispositif expérimental se trouve dans une pièce dédiée à l'imagerie de la surface oculaire. Cette méthode est non invasive. L'examen complet prend 5 min maximum par animal (voir Figure 8).

Sur les images obtenues pour les différents groupes, représentées sur les figures 1 à 7, il est constaté que les cornées des animaux ayant reçu une instillation biquotidienne de composé 1 (10 mM) pendant 5 jours consécutifs sont moins inflammatoires comparées à celles des animaux ayant reçu du PBS 1x.

### Exemple 2

L'objectif de cet exemple est de démontrer l'effet antiinflammatoire des composés de formule (I) telle que définie ci-avant et dans la revendication 1, et plus particulièrement du **composé 1,** deux marqueurs liés à cet état inflammatoire : l'ATF3 et le Iba1 (tel que décrit par Launay et al. dans Neurobiol. Dis., 88 (2016), 16-28) ont été quantifiés, dans le ganglion trigéminé, par immuno- histologie, dans le modèle de kératite inflammatoire induite par le LPS chez la souris tel que décrit dans l'exemple 1.

L'ATF3 (activating transcription facteur 3) est un facteur de transcription de la famille des ATF qui module la réponse inflammatoire de plusieurs maladies. Dans les conditions physiologiques, les neurones sensoriels primaires expriment très peu ATF3. Une augmentation du nombre de neurones positifs pour ATF-3 souligne alors une souffrance neuronale lié à un processus inflammatoire.

De la même manière, l'Iba1 (pour ionized calcium-binding adapter molécule 1) est exprimée dans les cellules microgliales activées par la présence de macrophages et donc par un processus inflammatoire.

### 1. Études immuno-histologiques

### Préparation des tissus

Deux heures après la dernière instillation de LPS, un mélange de 300 µL de kétamine 1000 U (100 mg / kg de poids corporel) et xylazine (10 mg / kg de poids corporel, Virbac, France) a été injecté par voie intrapéritonéale pour profondément anesthésier les animaux. Après la fixation, les ganglions trigéminés et les yeux ont été soigneusement disséqués et post-fixés 48 h dans le même mélange fixateur. Des sections (40 µm) du complexe du sous-nucléaire trigéminé (noyau trigéminé, TG) ont été réalisées en utilisant un vibratome (Leica Microsystems, Allemagne). Les TG ont été placés dans une solution à 10% de saccharose dans 1 x PBS (pendant la nuit), puis dans une solution à 30% de saccharose avant congélation à -20 °C dans un mélange de 7,5% de gélatine et 10% de saccharose. Des sections (14 µm) ont été effectuées au cryostat pour les TG et les yeux et conserver à -20 °C.

### Marquage par immunofluorescence

Après trois lavages dans 1 × PBS, les sections TG ont été placées dans une solution de fixation contenant 3% de sérum de cheval normal et 0,1% de triton X-100 pendant 2 h, puis incubé avec un anticorps primaire à 4 ° C pendant 48 h.

Les anticorps utilisés dans cette étude sont l'anti-ATF3 de lapin (Sigma-Aldrich: HPA001562, 1/500) et l' anti Iba1 de chèvre (Abcam: ab5076, 1/500). ATF3 a été amplifié en utilisant de la biotine de cheval conjugué à un anticorps de lapin (1/500; Vector Laboratories) pendant 1 h et finalement révélé par incubation avec la streptavidine-Alexa Fluor 488/555 (1/500; Invitrogen). Iba1 a été révélé à l'aide de la streptavidine Alexa Fluor 594 d'âne conjugué à l'anti-chèvre (1/500; Invitrogen) pendant 1 h.

Les sections TG ont été traitées en utilisant la peroxydase avidine-biotine (Vectastain ABC Kit, Vector Laboratories) pour l'immuno- réactivité ATF3 et Iba1. Les sections TG ont été incubées avec une solution à 3% de H₂O₂ pendant 20 min. Elles ont été rincées trois fois par 1 × PBS puis incubées avec 3% de NHS pendant 2 h à température ambiante avant incubation avec les anticorps primaires pendant la nuit à 4 °C. Les sections ont été rincées trois fois dans 1 x PBS, incubées pendant 1 h température ambiante avec l'IgG anti-lapin biotinylée secondaire (1/500 ; Vector). Les sections sont rincées trois fois dans 1 × PBS, incubées dans une solution contenant le complexe Avidine Biotin Peroxidase (Elite, Vector) pendant 1 h, puis rincé trois fois avec 1 x PBS.

### 2. Microscopie et quantification de l'étiquetage immunohistochimique

### Appareils microscopiques

Les cellules et les sections de tissu ont été examinées soit avec un microscope Zeiss M1 à épifluorescence ou avec un microscope confocal de balayage laser Olympus FV1000. Le microscope épifluorescence (Axio Imager M1; Carl Zeiss) est équipé d'un appareil photo numérique (Axio Cam HRC, Carl Zeiss) et du logiciel d'acquisition d'image (Zen; Carl Zeiss).

Le microscope confocal (Olympus FV1000) est équipé d'un laser à ion d'argon (488 nm) et à diodes laser (405 et 559 nm). Les images ont été acquises séquentiellement pour réduire les problèmes de diaphonie à l'excitation et à l'émission. La taille a été définie selon le théorème d'échantillonnage Nyquist-Shannon (1024 * 1024 pixels). Les objectifs utilisés étaient des objectifs Olympus PlanApo (10 / 0,40 NA) ou PlanApoN (60 / 1,42 NA, à immersion d'huile). Les sections de tissu ont également été scannées avec le scanner numérique Nanozoomer 2.0-HT (C9600, Hamamatsu Photonics), équipé du 3-CCD TDI caméra (Hamamatsu Photonics). Les images TIFF ont ainsi été obtenues. Les microscopes ont été calibrés avec les groupes contrôle.

### 3. Résultats

Les résultats obtenus sont reportés dans les figures 9 et 10.

Il apparaît clairement que l'instillation biquotidienne à 10 mM, pendant 5 jours par le composé 1 (PL265), dans un modèle de kératite inflammatoire induite par le LPS chez la souris permet de diminuer de manière importante l'expression de ATF3 et de Iba1 par rapport à une instillation identique avec du PBS et donc de démontrer une inflammation moins importante.

## Revendications

1. Composés répondant à la formule (I) ou un sel pharmaceutiquement acceptable de ces composés pour leur utilisation dans le traitement et/ou la prévention de l'inflammation oculaire, de préférence chez l'homme:
(I) R₁-NH-CH(R₂)-P(=O)(OH)-CH₂-C(R₃)(R₄)-CONH-C(R₅)(R₆)-COOR₇
dans laquelle :
R₁ représente
- un hydrogène
- un groupement (acyloxy)alkyl carbamate -C(=O)-O-C(R)(R')-OC(=O)-R" dans lequel R et R' représentent, indépendamment l'un de l'autre, un hydrogène, un groupement alkyle et R" représente un groupement alkyle,
R₂ représente :
- une chaîne hydrocarbonée, saturée ou insaturée, linéaire ou ramifiée comportant de 1 à 6 atomes de carbone,
R₃ et R₄ représentent indépendamment l'un de l'autre:
- un hydrogène
- un groupement phényle ou benzyle, éventuellement substitué sur le noyau phényle par :
* 1 à 5 atomes d'halogènes notamment le fluor ou le brome
* un radical OH, SH, OR" ou SR", R" ayant la même définition que précédemment
* un groupement amino éventuellement mono- ou di-substitué par un groupement aliphatique, cyclique ou linéaire, de 1 à 6 atomes de carbone
* un groupement trifluorométhyle
* un groupement aromatique ou hétéroaromatique à 5 ou 6 atomes
- un groupement hétéroaromatique à 5 ou 6 atomes, contenant 1 ou 2 hétéroatome(s) pris parmi l'oxygène, l'azote ou le soufre, les atomes de soufre et d'azote pouvant être oxydés sous forme de S-oxyde ou de N-oxyde
- un méthylène substitué par un hétérocycle à 5 ou 6 atomes, aromatique ou saturé, l'hétéroatome étant un oxygène, un azote ou un soufre, les atomes d'azote et de soufre pouvant être oxydés sous forme N-oxyde ou de S-oxyde
R₃ et R₄ ne représentent pas simultanément un atome d'hydrogène,
R₅ et R₆ représentent indépendamment l'un de l'autre
- un atome d'hydrogène
- une chaîne hydrocarbonée saturée ou insaturée, linéaire ou ramifiée de 1 à 6 atomes de carbone,
R₇ représente
- un hydrogène
- un radical CH₂COOR‴ ou CH(CH₃)COOR‴, R‴ représentant
*une chaîne hydrocarbonée saturée de 1 à 6 atomes de carbone, éventuellement substituée par un groupement alkoxy de C₁ à C₃,
*un groupement cycloalkyle de C₅ à C₈
*un groupement phényle, benzyle, hétéroaromatique ou alkyl hétéroaromatique
- un groupement CH(R)O-C(O)OR' ou CH(R)OC(O)R' dans lesquels R et R' ont les mêmes définitions que précédemment.

2. Composé pour son utilisation selon la revendication 1, pour améliorer le processus de guérison oculaire et/ou de cicatrisation oculaire.

3. Composé pour son utilisation selon la revendication 1 ou 2, pour prévenir la néovascularisation, pour prévenir l'apparition d'une kératopathie toxique, de problèmes au niveau de l'épithélium ou de cicatrices permanentes, ou pour prévenir ou traiter l'inflammation oculaire liée à une kératite, une sclérite, une épisclérite, une uvéite, une cataracte, une synéchie, un oedème maculaire, un décollement de la rétine, une hypertension oculaire et la dégénérescence du nerf optique causée par le glaucome.

4. Composé pour son utilisation selon l'une quelconque des revendications précédentes, **caractérisé en ce que** R₁ représente un groupement (acyloxy)alkyl carbamate -C(=O)-OC(R)(R')-OC(=O)-R".

5. Composé pour son utilisation selon l'une quelconque des revendications précédentes, **caractérisé en ce que** R₁ représente un groupement-C(=O)-O-CHMe-OC(=O)-CHMe₂.

6. Composé pour son utilisation selon l'une quelconque des revendications précédentes, **caractérisé en ce que** R₃ représente un atome d'hydrogène.

7. Composé pour son utilisation selon l'une quelconque des revendications précédentes, **caractérisé en ce que** R₄ représente groupe benzyle éventuellement substitué par 1 à 5 atome(s) d'halogène tel(s) que le fluor ou le brome, un phényle ou un groupement hétéroaromatique à 5 ou 6 chaînons, de préférence R₄ représente groupe benzyle éventuellement substitué par un phényle.

8. Composé pour son utilisation selon l'une quelconque des revendications précédentes, **caractérisé en ce que** R₅ représente un atome d'hydrogène.

9. Composé pour son utilisation selon l'une quelconque des revendications précédentes, **caractérisé en ce que** R₆ représente un groupement alkyle tel qu'un groupement méthyle.

10. Composé pour son utilisation selon l'une quelconque des revendications précédentes, **caractérisé en ce que** R₇ représente un atome d'hydrogène.

11. Composé pour son utilisation selon l'une quelconque des revendications précédentes, **caractérisé en ce qu'**il est choisi parmi les composés suivants :
Ester benzylique de l'acide 2-(2-Biphényl-4-ylméthyl-3-{hydroxy-[1-(1-isobutyryloxy-éthoxycarbonylamino)-éthyl]-phosphinoyl}-propionylamino)-propionique
Acide 2-(2-Biphényl-4-ylméthyl-3-{hydroxy-[1-(1-isobutyryloxy-éthoxycarbonylamino)-éthyl]-phosphinoyl}-propionylamino)-propionique
Ester éthylique de l'acide 2-(2-Biphényl-4-ylméthyl-3-{hydroxy-[1-(1-isobutyryloxy-éthoxycarbonylamino)-éthyl]-phosphinoyl}-propionylamino)-propionique
Ester éthoxycarbonyloxy de l'acide 2-(2-Biphényl-4-ylméthyl-3-{hydroxy-[1-(1-isobutyryloxy-éthoxycarbonylamino)-éthyl]-phosphinoyl}-propionylamino)-propionique
Ester benzylique de l'acide 2-(2-(4-Bromo-benzyl)-3-{hydroxy-[1-(1-isobutyryloxy-éthoxycarbonylamino)-éthyl]-phosphinoyl}-propionylamino)-propionique
Acide 2-(2-(4-Bromo-benzyl)-3-{hydroxy-[1-(1-isobutyryloxy-éthoxycarbonylamino)-éthyl]-phosphinoyl}-propionylamino)-propionique
Ester benzylique de l'acide 2-(2-Biphényl-4-ylméthyl-3-{hydroxy-[1-(amino)-éthyl]-phosphinoyl}-propionylamino)-propionique
Acide 2-(2-Biphényl-4-ylméthyl-3-{hydroxy-[1-(amino)-éthyl]-phosphinoyl}-propionylamino)-propionique
Ester éthylique de l'acide 2-(2-Biphényl-4-ylméthyl-3-{hydroxy-[1-(1amino)-éthyl]-phosphinoyl}-propionylamino)-propionique
Ester éthoxycarbonyloxy de l'acide 2-(2-Biphényl-4-ylméthyl-3-{hydroxy-[1-(amino)-éthyl]-phosphinoyl}-propionylamino)-propionique
Ester benzylique de l'acide 2-(2-(4-Bromo-benzyl)-3-{hydroxy-[1-(amino)-éthyl]-phosphinoyl}-propionylamino)-propionique
Acide 2-(2-(4-Bromo-benzyl)-3-{hydroxy-[1-(amino)-éthyl]-phosphinoyl}-propionylamino)-propionique
Ester éthylique de l'acide 2-(2-(4-Bromo-benzyl)-3-{hydroxy-[1-(amino)-éthyl]-phosphinoyl}-propionylamino)-propionique.

12. Composition ophtalmique comprenant un composé de formule (I) telle que définie dans l'une quelconque des revendications 1 à 11, en particulier collyre, pommade ophtalmique, gel ophtalmique, ou insert ophtalmique, pour son utilisation dans le traitement et/ou la prévention de l'inflammation oculaire.

13. Composition pour utilisation selon la revendication 12, **caractérisée en ce qu'**elle comprend de 0,01% à 3% en poids par volume dudit composé de formule (I), avantageusement de 0,1% à 1% en poids par volume.

14. Composition pour utilisation selon la revendication 12 ou 13, **caractérisée en ce qu'**elle est une solution aqueuse, ayant avantageusement un pH allant de 5,0 à 8, notamment entre 5,5 et 8,0.

15. Composition pour utilisation selon la revendication 12 à 14, **caractérisée en ce qu'**elle comprend en outre une cyclodextrine, telle que l'hydroxypropyl béta-cyclodextrine ou bien encore la sulfobutyl ether béta-cyclodextrine, ou du sodium de polystyrène sulfonate.

## Patentansprüche

1. Verbindungen, die der Formel (I) entsprechen, oder ein pharmazeutisch akzeptables Salz dieser Verbindungen für ihre Verwendung zur Behandlung und/oder Vorbeugung von Augenentzündungen, vorzugsweise beim Menschen:
(I) R₁-NH-CH(R₂)-P(=O)(OH) -CH₂-C(R₃)(R₄)-CONH-C (R₅)(R₆)-COOR₇
wobei:
R₁ darstellt
- Wasserstoff,
- eine (Acyloxy)alkylcarbamatgruppe - C (=O)-O-C (R) (R' ) -OC (=O)-R", wobei R und R' unabhängig voneinander Wasserstoff, eine Alkylgruppe darstellen und R" eine Alkylgruppe darstellt,
R₂ darstellt:
- eine gesättigte oder ungesättigte, lineare oder verzweigte Kohlenwasserstoffkette mit 1 bis 6 Kohlenstoffatomen,
R₃ und R₄ unabhängig voneinander darstellen:
- Wasserstoff,
- eine Phenyl- oder Benzylgruppe, eventuell substituiert auf dem Phenylkern durch:
* 1 bis 5 Halogenatome, insbesondere Fluor oder Brom,
* ein OH-, SH-, OR"- oder SR"-Radikal, wobei R" dieselbe Definition wie zuvor hat,
* eine Aminogruppe, gegebenenfalls mono- oder disubstituiert durch eine aliphatische, zyklische oder lineare Gruppe, mit 1 bis 6 Kohlenstoffatomen,
* eine Trifluormethyl-Gruppe,
* eine aromatische oder heteroaromatische Gruppe mit 5 oder 6 Atomen,
- eine heteroaromatische Gruppe mit 5 oder 6 Atomen, die 1 oder 2 Heteroatome enthält, die aus Sauerstoff, Stickstoff oder Schwefel ausgewählt sind, wobei die Schwefel- und Stickstoffatome in Form von S-Oxid oder N-Oxid oxidiert sein können,
- ein Methylen, substituiert durch einen Heterozyklus mit 5 oder 6 Atomen, aromatisch oder gesättigt, wobei das Heteroatom Sauerstoff, Stickstoff oder Schwefel ist, wobei die Stickstoff- und Schwefelatome in Form von N-Oxid oder S-Oxid oxidiert sein können,
R₃ und R₄ nicht gleichzeitig ein Wasserstoffatom darstellen,
R₅ und R₆ unabhängig voneinander darstellen
- ein Wasserstoffatom,
- eine gesättigte oder ungesättigte, lineare oder verzweigte Kohlenwasserstoffkette mit 1 bis 6 Kohlenstoffatomen,
R₇ darstellt:
- Wasserstoff,
- ein CH₂COOR'"- oder CH(CH₃)COOR'"-Radikal, wobei R'" darstellt:
* eine gesättigte Kohlenwasserstoffkette mit 1 bis 6 Kohlenstoffatomen, eventuell substituiert durch eine C₁-C₃-Alkoxygruppe,
* eine C₅-C₈-Cycloalkylgruppe,
* eine Phenyl-, Benzyl-, heteroaromatische oder alkylheteroaromatische Gruppe,
- eine CH(R)O-C(O)OR'- oder CH(R)OC(O)R'-Gruppe, wobei R und R' die gleichen Definitionen wie zuvor haben.

2. Verbindung für ihre Verwendung nach Anspruch 1 zur Verbesserung des Prozesses der Augengenesung und/oder Augenheilung.

3. Verbindung für ihre Verwendung nach Anspruch 1 oder 2 zur Vorbeugung einer Neovaskularisierung, zur Vorbeugung des Auftretens einer toxischen Keratopathie, von Problemen im Bereich des Epithels oder dauerhaften Narben oder zur Vorbeugung oder Behandlung einer Augenentzündungen in Vebindung mit einer Keratitis, Skleritis, Episkleritis, Uveitis, einem Katarakt, einer Synechie, einem Makulaödem, einer Netzhautablösung, einer Hypertonie der Augen und der Degeneration des Sehnervs aufgrund von Glaukom.

4. Verbindung für ihre Verwendung nach einem der vorhergehenden Ansprüche, **dadurch gekennzeichnet, dass** R1 eine (Acyloxy)alkylcarbamatgruppe -C(=O)-O-C(R)-OC(=O)-R" darstellt.

5. Verbindung für ihre Verwendung nach einem der vorhergehenden Ansprüche, **dadurch gekennzeichnet, dass** R1 eine Gruppe -C(=O)-O-CHMe-OC(=O)-CHMe₂ darstellt.

6. Verbindung für ihre Verwendung nach einem der vorhergehenden Ansprüche, **dadurch gekennzeichnet, dass** R₃ ein Wasserstoffatom darstellt.

7. Verbindung für ihre Verwendung nach einem der vorhergehenden Ansprüche, **dadurch gekennzeichnet, dass** R₄ eine Benzylgruppe, eventuell durch 1 bis 5 Halogenatome wie Fluor oder Brom substituiert, ein Phenyl oder eine heteroaromatische Gruppe mit 5 oder 6 Gliedern darstellt, wobei R₄ vorzugsweise eine Benzylgruppe, eventuell substituiert durch ein Phenyl, darstellt.

8. Verbindung für ihre Verwendung nach einem der vorhergehenden Ansprüche, **dadurch gekennzeichnet, dass** R₅ ein Wasserstoffatom darstellt.

9. Verbindung für ihre Verwendung nach einem der vorhergehenden Ansprüche, **dadurch gekennzeichnet, dass** R₆ eine Alkylgruppe wie eine Methylgruppe darstellt.

10. Verbindung für ihre Verwendung nach einem der vorhergehenden Ansprüche, **dadurch gekennzeichnet, dass** R₇ ein Wasserstoffatom darstellt.

11. Verbindung für ihre Verwendung nach einem der vorhergehenden Ansprüche, **dadurch gekennzeichnet, dass** sie aus den folgenden Verbindungen ausgewählt ist:
2-(2-Biphenyl-4-ylmethyl-3-{hydroxy-[1-(1-isobutyryloxy-ethoxycarbonylamino)-ethyl]-phosphinoyl}-propionylamino)-propionsäurebenzylester
2-(2-Biphenyl-4-ylmethyl-3-{hydroxy-[1-(1-isobutyryloxy-ethoxycarbonylamino)-ethyl]-phosphinoyl}-propionylamino)-propionsäure
2-(2-Biphenyl-4-ylmethyl-3-{hydroxy-[1-(1-isobutyryloxy-ethoxycarbonylamino)-ethyl]-phosphinoyl}-propionylamino)-propionsäureethylester
2-(2-Biphenyl-4-ylmethyl-3-{hydroxy-[1-(1-isobutyryloxy-ethoxycarbonylamino)-ethyl]-phosphinoyl}-propionylamino)-propionsäureethoxycarbonyloxyester
2-(2-(4-Bromo-benzyl)-3-{hydroxy-[1-(1-isobutyryloxy-ethoxycarbonylamino)ethyl]-phosphinoyl}-propionylamino)-propionsäurebenzylester
2-(2-(4-Bromo-benzyl)-3-{hydroxy-[1-(1-isobutyryloxy-ethoxycarbonylamino)-ethyl]-phosphinoyl}-propionylamino)-propionsäure
2-(2-Biphenyl-4-ylmethyl-3-{hydroxy-[1-(amino)ethyl]-phosphinoyl}-propionylamino)-propionsäurebenzylester
2-(2-Biphenyl-4-ylmethyl-3-{hydroxy-[1-(amino)ethyl]-phosphinoyl}-propionylamino)-propionsäure
2-(2-Biphenyl-4-ylmethyl-3-{hydroxy-[1-(1amino)-ethyl]-phosphinoyl}-propionylamino)-propionsäureethylester
2-(2-Biphenyl-4-ylmethyl-3-{hydroxy-[1-(amino)ethyl]-phosphinoyl}-propionylamino)-propionsäureethoxycarbonyloxyester
2-(2-(4-Bromo-benzyl)-3-{hydroxy-[1-(amino)-ethyl]-phosphinoyl}-propionylamino)-propionsäurebenzylester
2-(2-(4-Bromo-benzyl)-3-{hydroxy-[1-(amino)-ethyl]-phosphinoyl}-propionylamino)-propionsäure
2-(2-(4-Bromo-benzyl)-3-{hydroxy-[1-(amino)-ethyl]-phosphinoyl}-propionylamino)-propionsäureethylester.

12. Ophtalmologische Zusammensetzung, die eine Verbindung der wie in einem der Ansprüche 1 bis 11 definierten Formel (I) umfasst, insbesondere Augentropfen, Augensalbe, Augengel oder ophthalmische Einlage, für ihre Verwendung zur Behandlung und/oder Vorbeugung von Augenentzündungen.

13. Zusammensetzung zur Verwendung nach Anspruch 12, **dadurch gekennzeichnet, dass** sie 0,01 bis 3 Gew.-% pro Volumen der Verbindung der Formel (I), vorteilhafterweise 0,1 bis 1 Gew.-% je Volumen, umfasst.

14. Zusammensetzung zur Verwendung nach Anspruch 12 oder 13, **dadurch gekennzeichnet, dass** sie eine wässrige Lösung ist, die vorteilhafterweise einen pH-Wert von 5,0 bis 8, insbesondere zwischen 5,5 und 8,0, aufweist.

15. Zusammensetzung zur Verwendung nach Anspruch 12 bis 14, **dadurch gekennzeichnet, dass** sie ferner ein Cyclodextrin wie Hydroxypropyl Beta-Cyclodextrin oder auch Sulfobutyl Ether Beta-Cyclodextrin oder Polystyrolsulfonat-Natrium umfasst.

## Claims

1. Compounds having the formula (I) or a pharmaceutically acceptable salt thereof for use in the treatment and/or prevention of eye inflammation, preferably in humans:
(I) R₁-NH-CH(R₂)-P (=O)(OH) -CH₂-C(R₃)(R₄)-CONH-C (R₅)(R₆)-COOR₇
wherein:
R₁ represents
- hydrogen,
- an (acyloxy)alkyl carbamate -C(=O)-O-C(R) (R') -OC(=O)-R" group in which R and R' independently represent hydrogen, an alkyl group and R" represents an alkyl group,
R₂ represents:
- a linear or branched saturated or unsaturated hydrocarbon chain having from 1 to 6 carbon atoms,
R₃ and R₄ represent independently of each other:
- hydrogen,
- a phenyl or benzyl group, possibly substituted on the phenyl ring with:
* 1 to 5 halogen atoms especially fluorine or bromine,
* an OH, SH, OR" OR SR" radical, R" having the same definition as previously,
* an amino group optionally mono- or disubstituted with an aliphatic, cyclic or linear group, having from 1 to 6 carbon atoms,
* a trifluoromethyl group,
* an aromatic or heteroaromatic group with 5 or 6 atoms,
- a heteroaromatic group with 5 or 6 atoms, containing 1 or 2 heteroatom(s) selected from oxygen, nitrogen or sulphur, the sulphur and nitrogen atoms possibly being oxidised in the form of S-oxide or N-oxide,
- a methylene substituted with a 5- or 6-atom heterocycle, aromatic or saturated, the heteroatom being oxygen, nitrogen or sulphur, the nitrogen and sulphur atoms being oxidizable in N-oxide or S-oxide form,
R₃ and R₄ do not simultaneously represent a hydrogen atom,
R₅ and R₆ represent independently of each other
- a hydrogen atom
- a saturated or unsaturated, linear or branched hydrocarbon chain having from 1 to 6 carbon atoms,
R₇ represents
- hydrogen
- a CH₂ COOR'" or CH(CH₃) COOR' "radical, R'" representing
* a saturated hydrocarbon chain having from 1 to 6 carbon atoms, optionally substituted with a C₁-C₃ alkoxy group,
* a C5-C8 cycloalkyl group,
* a phenyl, benzyl, heteroaromatic or alkyl heteroaromatic group,
- a CH(R)O-C(O)OR' or CH(R)OC(O)R' group in which R and R' have the same definitions as previously.

2. The compound for use according to claim 1, for improving the process of ocular recovery and/or ocular healing.

3. The compound for use according to claim 1 or 2, for preventing neovascularisation, for preventing the occurrence of toxic keratopathy, epithelium problems, or permanent scarring, or for preventing or treating ocular inflammation related to keratitis, scleritis, episcleritis, uveitis, cataract, synechia, macular oedema, retinal detachment, ocular hypertension, and optic nerve degeneration caused by glaucoma.

4. The compound for use according to any one of the preceding claims, **characterised in that** R₁ represents a (acyloxy)alkyl carbamate -C(=O)-O-C(R)(R' )-OC(=O)-R" group.

5. The compound for use according to any one of the preceding claims, **characterised in that** R₁ represents a-C(=O)-O-CHMe-OC(=O)-CHMe₂ group.

6. The compound for use according to any one of the preceding claims, **characterised in that** R₃ represents a hydrogen atom.

7. The compound for use according to any one of the preceding claims, **characterised in that** R₄ represents a benzyl group optionally substituted with 1 to 5 halogen atom(s) such as fluorine or bromine, a phenyl or a heteroaromatic 5- or 6-member group, preferably R₄ represents a benzyl group optionally substituted with a phenyl.

8. The compound for use according to any one of the preceding claims, **characterised in that** R₅ represents a hydrogen atom.

9. The compound for use according to any one of the preceding claims, **characterised in that** R₆ represents an alkyl group such as a methyl group.

10. The compound for use according to any one of the preceding claims, **characterised in that** R₇ represents a hydrogen atom.

11. The compound for use according to any one of the preceding claims, **characterised in that** it is selected from the following compounds:
2-(2-Biphenyl-4-ylmethyl-3-{hydroxy-[1-(1-isobutyryloxy- ethoxycarbonylamino)-ethyl]-phosphinoyl}-propionylamino)-propionic acid benzyl ester
2-(2-Biphenyl-4-ylmethyl-3-{hydroxy-[1-(1-isobutyryloxy-ethoxycarbonylamino)-ethyl]-phosphinoyl}-propionylamino)-propionic acid
2-(2-Biphenyl-4-ylmethyl-3-{hydroxy-[1-(1-isobutyryloxy- ethoxycarbonylamino)-ethyl]-phosphinoyl}-propionylamino)-propionic acid ethyl ester
2-(2-Biphenyl-4-ylmethyl-3-{hydroxy-[1-(1-isobutyryloxy-ethoxycarbonylamino)-ethyl]-phosphinoyl}-propionylamino)-propionic acid ethoxycarbonyloxy ester
2-(2-(4-Bromo-benzyl)-3-{hydroxy-[1-(1-isobutyryloxy- ethoxycarbonylamino)-ethyl]-phosphinoyl}-propionylamino)-propionic acid benzyl ester
2-(2-(4-Bromo-benzyl)-3-{hydroxy-[1 -(1 - isobutyryloxy-ethoxycarbonylamino)-ethyl]-phosphinoyl}-propionylamino)-propionic acid
2-(2-Biphenyl-4-ylmethyl-3-{hydroxy-[1-(amino)-ethyl]- phosphinoyl}-propionylamino)-propionic acid benzyl ester
2-(2-Biphenyl-4-ylmethyl-3-{hydroxy-[1-(amino)-ethyl]-phosphinoyl}-propionylamino)-propionic acid
2-(2-Biphenyl-4-ylmethyl-3-{hydroxy-[1-(1amino)-ethyl]- phosphinoyl}-propionylamino)-propionic acid ethyl ester
2-(2-Biphenyl-4-ylmethyl-3-{hydroxy-[1-(amino)-ethyl]- phosphinoyl}-propionylamino)-propionic acid ethoxycarbonyloxy ester
2-(2-(4-Bromo-benzyl)-3-{hydroxy-[1-(amino)-ethyl]- phosphinoyl}-propionylamino)-propionic acid benzyl ester
2-(2-(4-Bromo-benzyl)-3-{hydroxy-[1-(amino)-ethyl]-phosphinoyl}-propionylamino)-propionic acid
2-(2-(4-Bromo-benzyl)-3-{hydroxy-[1-(amino)-ethyl]- phosphinoyl}-propionylamino)-propionic acid ethl ester.

12. An ophthalmic composition comprising a compound of the formula (I) as defined in any one of claims 1 to 11, in particular eye drops, ophthalmic ointment, ophthalmic gel, or prescription insert, for use in the treatment and/or prevention of eye inflammation.

13. The composition for use according to claim 12, **characterised in that** it comprises from 0.01% to 3% by weight per volume of said compound of formula (I), advantageously from 0.1% to 1% by weight per volume.

14. The composition for use according to claim 12 or 13, **characterised in that** it is an aqueous solution, advantageously having a pH ranging from 5.0 to 8, especially from 5.5 to 8.0.

15. The composition for use according to claim 12 to 14, **characterised in that** it further comprises cyclodextrin, such as hydroxypropyl beta-cyclodextrin or even sulfobutyl ether beta-cyclodextrin, or sodium polystyrene sulfonate.
